Europäisches Patentamt

European Patent Office

Office européen des brevets

(19)

(11) **EP 1 106 592 A1**

(12) **EUROPEAN PATENT APPLICATION**
published in accordance with Art. 158(3) EPC

(43) Date of publication:
**13.06.2001 Bulletin 2001/24**

(51) Int Cl.⁷: **C07C 6/12**, C07C 15/06,
C07C 15/08, B01J 29/16,
B01J 29/26

(21) Application number: **00938465.2**

(22) Date of filing: **21.06.2000**

(86) International application number:
**PCT/CN00/00168**

(87) International publication number:
**WO 01/00547 (04.01.2001 Gazette 2001/01)**

(84) Designated Contracting States:
**AT BE CH CY DE DK ES FI FR GB GR IE IT LI LU
MC NL PT SE**

(30) Priority: **24.06.1999 CN 99113812**

(71) Applicant: **CHINA PETRO-CHEMICAL
CORPORATION
Beijing 100029 (CN)**

(72) Inventors:
• **KONG, Dejin
  Pudong Shanghai 201208 (CN)**

• **CHENG, Wencai
  Pudong Shanghai 201208 (CN)**
• **LI, Huaying
  Pudong Shanghai 201208 (CN)**
• **ZOU, Wei
  Pudong Shanghai 201208 (CN)**
• **GUO, Hongli
  Pudong Shanghai 201208 (CN)**

(74) Representative: **Caldwell, Judith Margaret et al
David Keltie Associates,
12 New Fetter Lane
London EC4A 1AG (GB)**

(54) **A PROCESS FOR THE DISPROPORTION AND TRANSALKYLATION OF TOLUENE AND C9+ HEAVY AROMATIC HYDROCARBONS, AND ITS CATALYST**

(57)    The present invention relates to a process of the disproportionation and transalkylation of toluene and heavy aromatic hydrocarbons of nine and more than nine carbon atoms and also to a catalyst used in the process. It can be mainly used to overcome the disadvantages of the known processes in the prior arts. The said disadvantages include that the processing quantity of heavy aromatic hydrocarbons is lower; only the dealkylation and lightening are carried out in the known processes in the prior arts resulting in a large amount of non-aromatic light hydrocarbons and the stringent requirements on the composition of feedstock is needed. The present invention can be used to effectively carry out the disproportionation and transalkylation of toluene and heavy aromatic hydrocarbons of nine and more than nine carbon atoms by using the catalyst made of zeolite and molybdenum oxide and other active components supported on the zeolite so as to form a large quantity of benzene and xylene products. In addition, the catalyst of the present invention is of such characters as high catalytic activity, a great processing capacity of heavy aromatic hydrocarbons of ten and more than ten carbon atoms and high selectivity to desired products of benzene and aromatics of eight carbon atoms, so the present invention can be successfully used in the industrial production.

EP 1 106 592 A1

**Description**

FIELD OF THE INVENTION

The present invention relates to a process and a catalyst for the disproportionation and transalkylation of toluene and heavy aromatic hydrocarbons of nine and more than nine carbon atoms. In particular, the present invention relates to a process and a catalyst for preparing benzene and aromatics of eight carbon atoms through the disproportionation and transalkylation of toluene and the above-mentioned heavy aromatic hydrocarbons.

BACKGROUND OF THE INVENTION

p-xylene, a kind of aromatic hydrocarbons of eight carbon atoms-$C_8A$, is one of the basic organic chemical materials in the petrochemical industry. It has been widely used in many manufacturing fields of chemical products such as chemical fibers, synthetic resins, pesticides, pharmaceuticals, plastics etc. The typical known process for preparing p-xylene is as follows: firstly, $C_8A$ is produced by catalytic reforming of naphtha; then the p-xylene is separated out from the mixture of its isomers having boiling point close to that of p-xylene through multiplestage cryogenic crystalline separation or molecular sieve analogue moving bed adsorption separation. While the o- and m-xylene and ethylbenzene are usually treated to be isomerized to p-xylene. In order to increase the yield of p-xylene, the disproportionation of toluene or the disproportionation and transalkylation of tolulene and heavy aromatic hydrocarbons of nine and more than nine carbon atoms, namely $C_9^+A$, are properly used to form benzene and $C_8A$, so that the yield of p-xylene can be effectively improved as the increase of $C_8A$ yield.

Aromatic hydrocarbons of nine carbon atoms ($C_9A$) and aromatic hydrocarbons consisting of ten and more than ten carbon atoms, namely $C_{10}^+A$, are often generally referred to as heavy aromatic hydrocarbons, and also named as $C_9^+A$. In fact, $C_{10}^+A$ always includes non-aromatics, methyl-naphthalenes and fused ring compounds etc.. The heavy aromatic hydrocarbons are mainly derived from the side products of light-oil cracking process for producing ethylene; and from aromatics produced by catalytic reforming in refinery; as well as from side products of toluene disproportionation and transalkylation process. As the quantity of heavy aromatic hydrocarbons greatly increases, the comprehensive utilization of heavy aromatic hydrocarbons becomes a great concerned issue. At present, there are some known mature processes for processing and utilizing $C_9A$, for example, $C_9A$ has been widely used as the feedstock in the process of disproportionation and transalkylation of toluene and $C_9A$ to produce benzene and $C_8A$. The composition of $C_{10}^+A$ fraction in the side products derived from the known aromatics complex is very complicated, besides 5-20%(weight) of $C_9A$ such as methylethylbenzene, trimethylbenzene etc. and indane(IND). $C_{10}^+A$ fraction also contains dozens kinds of components such as heavy aromatic hydrocarbons of ten carbon atoms ($C_{10}A$), and heavy aromatic hydrocarbons mainly consisting of eleven and more than eleven carbon atoms ($C_{11}^+A$), for example, tetramethylbenzene, dimethylethyl-benzene, diethyl-benzene, methyl-naphthalene, dimethyl-naphthalene etc. Therefore, $C_{10}^+A$ fraction is of little use at present due to its complicated composition and high boiling points. On the other hand, these components are also not suitable to be used as an additive in gasoline or diesel. Only some of them may be used as solvent oil or as the feedstock for separating durene, and most of them are used as burning fuel, so as to cause the waste of resources. According to the statistics, an aromatics complex of 225 thousand tons of p-xylene output per year may produce about 10-30 thousand tons of $C_{10}^+A$ each year, depending on different feedstock oils and different processes. $C_{10}^+A$ is a valuable resource. Unfortunately, there is no any proper process in the prior arts to effectively process and utilize it in the aromatics complex.

Various relevant processes and catalysts were disclosed in the early issued patents and related references for improving the catalytic selectivity of desired products and running time in the process of the disproportionation and transalkylatoin of toluene and $C_9A$ by limiting strictly the amount of indane in the feedstock. Due to the fact that the boiling point of indane is between that of $C_9A$ and $C_{10}A$, $C_{10}A$ is unable to be utilized in processes in the prior arts. With the development of catalyst technology, it now becomes possible to improve the yield of desired products by using the feedstock into which a certain amount of $C_{10}A$ was added, so that it can make up the disadvantage of low yield caused by adopting high conversion and high space velocity which are often used to improve the yield of desired products. However, it is still impossible to effectively utilize the $C_{11}^+A$. The introduction of $C_{11}^+A$ into the feedstock will seriously affect the stability of the catalyst used, therefore, the process can not be operated under a stable condition. Due to the fact that $C_{10}A$ has very complicated composition and the wide distillation range, as well as the fact that the boiling points of some of components in $C_{10}A$ are higher than the boiling point of $C_{11}^+A$, the operating conditions of the heavy aromatic hydrocarbon column should be strictly controlled in the process of separating $C_{10}A$. Accordingly, in order to prevent $C_{11}^+A$ from being mixed into the reaction feedstock, in practice, only about 20-30% of $C_{10}A$ can be utilized and about 80-70% of $C_{10}A$ is discharged along with $C_{11}^+A$ from the bottom of the heavy aromatic hydrocarbon column.

Generally, typical $C_{10}^+A$ comprises about 60-65% of $C_{10}A$ and 40-35% of $C_{11}^+A$. It is noted that the utilization

of $C_{10}{}^+A$ will highly depend on whether the $C_{11}{}^+A$ can be utilized or not.

Since the expensive benzene and $C_8A$ have been widely used in the chemical industry, on the other hand, large amount of $C_{10}{}^+A$ still has not been fully used, processes for manufacturing benzene and $C_8A$ by the disproportionation and transalkylation of toluene and $C_9{}^+A$ are gradually concerned.

Three popular processes of producing benzene and $C_8A$ by the means of the disproportionation and transalkylation of toluene and/or $C_9{}^+A$ are known at present.

In the first said process, the benzene and $C_8A$ were obtained through the disproportionation and transalkylation of aromatic hydrocarbons wherein the mixture of toluene and a suitable amount of heavy aromatic hydrocarbons was used as feedstock. As matter of the fact, this process was a conventional technology known as Tatoray technology. Due to the fact that the heavy aromatic hydrocarbons used in such process contained 95%(wt) of $C_9A$, the cheaper $C_{10}{}^+A$ had not been effectively used in the practical industrial aromatic hydrocarbons complex. Although the feedstock used in such process could contain $C_{10}A$, $C_{11}{}^+A$ could not be processed. Furthermore the content of $C_{10}A$ must be strictly controlled and the total content of toluene and $C_9A$ was required to be at least 97% (wt) or above and the allowed content of $C_{10}A$ was only 3%(wt) or lower. It indicated that the normal operation of the process would be seriously affected if the content of $C_{10}A$ in the feedstock was higher than 3%(wt).

US Patent 4,172,813 disclosed a process in which $WO_3$-$MoO_3$-mordenite-$Al_2O_3$ (3% $WO_3$, 5% $MoO_3$, 60% mordenite and 40% $Al_2O_3$ by weight) was used as catalyst to make heavy reforming liquid undergo a selectively hydrodealkylation and transalkylation wherein the reaction temperature was from 315°C to 538°C and the reaction pressure was from 150Psig to 500Psig, the reaction feedstock contained 0.5% of non- aromatic hydrocarbons, 0.4% of $C_8A$, 28.3% of toluene, 46.6% of trimethylbenzene, 11.6% of methylethyl-benzene, 2.1% of indane and propyl-benzene, 10.1% of tetramathyl-benzene, and only 0.4% of $C_{11}{}^+A$. The desired products were mainly obtained by the means of forming light aromatics by dealkylation of heavy aromatic hydrocarbons. This said process had such disadvantages that a large amount of non-aromatics was formed in the process, as well as the yield of aromatic hydrocarbons was relatively lower.

In the second process, the desired products were obtained entirely by the means of lightening heavy aromatic hydrocarbons. For example, one such process disclosed in Japanese Patent 51-29131 wherein $MoO_3$-$NiO/Al_2O_3$ (13% weight of Mo, 5% weight of Ni) was used as catalyst, $C_9A$-$C_{10}{}^+A$ [a composition of 0.81% of benzene by weight, 0.26% of toluene by weight, 0.95% of $C_8A$ by weight, 80.96% of $C_9A$ by weight, 15.23% of $C_{10}{}^+A$ by weight] was used as feedstock. The process was carried out at 6MPa(G) and 550°C, the obtained products contained (by weight): 9.74% of benzene, 30.27% of toluene, 32.23% of $C_8A$ and 0.16% of non-aromatics. Although it was possible that higher content of $C_{10}{}^+A$ was processed in the process, the amount of light hydrocarbons was considerably raised due to the fact that the desired products were mainly obtained through the dealkylation of heavy aromatic hydrocarbons to form light hydrocarbons. Obviously, this process would cause great waste of resources because the light hydrocarbons generally were used as burning gas and the rest was transferred to torch assembly to be burned in the present unit.

In the third process, pure toluene as feedstock was used to selectively produce p-Xylene by the means of selective disproportionation of toluene. In this process, ZSM-5 zeolite was mainly used as active component in the catalyst. In recent years, it often could be seen in references such as US Patent 5,403,800(1995), US Patent 5,633,417 (1997). ZSM-5 zeolite catalyst with medium-size pore structure was mostly applied in the selective disproportionation of toluene.

All above-mentioned processes were carried out in fixed bed reactor in the presence of hydrogen. The presence of hydrogen was to prevent the catalysts from being coked, so as to prolong the running period of catalysts and to protect the catalysts.

Under the same operating conditions, the higher the content of $C_9{}^+A$, in particular the higher the content of $C_{10}{}^+A$, in feedstock was, the easier the catalysts were to be coked. Therefore, the content of $C_{10}{}^+A$ in the reaction feedstock was strictly limited in the conventional processes.

Accordingly, the traditional Tatoray process was often used in the most of present units in which toluene and $C_9A$ were used as the feedstock to produce benzene and $C_8A$ by the means of disproportionation and transalkylation. Generally, the said process was carried out in the fixed bed reactor in the presence of hydrogen and mordenite as catalyst by using toluene and $C_9A$ as the feedstock to form $C_6$-$C_{10}$ aromatics, $C_1$-$C_5$ alkanes and small amount of $C_{11}{}^+A$. The toluene and $C_9A$ separated out from the reaction effluents were circulated and merged with fresh toluene and $C_9A$ from outside battery, and the resultant mixture was used as the feedstock of reactor. Due to the limitation of catalyst performances, the $C_{10}A$ and $C_{11}{}^+A$ produced by unit itself were not able to enter into the circulating as a feedstock and were discharged from heavy aromatic hydrocarbon column. In addition, due to limitation of catalyst performances, the technical indexes of the process were lower, typical operating conditions were as follows: weight ratio of toluene to $C_9A$ in the reaction feedstock was from 80:20 to 60:40 by weight, the reaction pressure was 3.0 MPa (G), molecular ratio of hydrogen to aromatics was not less than 6, usually 8-10, weight hourly space velocity was 1.0-1.5$hr^{-1}$, usually 1.1-1.3$hr^{-1}$, the conversion of (toluene+$C_9A$) was within 40-43%, usually 40-41%, selectivity to (benzene+$C_8A$) was not higher than 94%.

Various issued patents and relevant references described the process of toluene disproportionation and transalkylation characterized in mordenite used as catalyst. For example, US Patents 2,795,629, 3,551,510, 3,701,813, and 3,729,521 described the catalysts for transalkylation, the composition of the reaction feedstock and the reaction condition. The issued US Patents 2,795,629, 3,780,122, and 3,849,340 described the catalysts used for transalkylation technology. Due to the limitation of the catalyst performances, the above-mentioned patents were not able to make full use of $C_9^+A$. Besides the yield of desired products was also relatively low, the economic and technical indexes were not satisfied.

US Patent 4,341,914 disclosed an improved Tatoray process. Although the $C_{10}A$ produced by the reaction itself could be partially used by being circulated and introduced into the reaction feedstock, it could only control the reaction of forming $C_{10}^+A$ thereby reduce the output of $C_{10}^+A$ and improve the yields of benzene and $C_8A$. In fact, the process could not make use of $C_{10}^+A$. In addition, due to the limitation of the catalyst performances, the feedstock used in the process must meet some requirements, for example, the content of indane in the feedstock should be strictly limited. Therefore the $C_{10}^+A$ could not be used as the reaction feedstock.

US Patent 4,665,258(1987) described a new improved process of the toluene disproportionation in which aluminum-lean mordenite was used as catalyst. The reaction was carried out under relatively stringent conditions. The ratio of silica to alumina in mordenite used as catalyst was more than 30, preferably 40-60. But its activity and stability were not satisfied. In addition, the use of $C_{10}^+A$ was not mentioned.

Japanese Patent 49-46295 disclosed a process for preparing a catalyst used to produce alkylbenzene. The said catalyst took mordenite as a main active component, and zirconium and other promoters selected from a group consisting of silver, bismuth, copper and antimony etc. were supported on the modenite. The said catalyst could be used in the disproportionation of aromatic hydrocarbons. But this patent was not involved with the processing of heavy aromatic hydrocarbons and the reaction performance for disproportionation and transalkylation of toluene and $C_9^+A$.

Chinese Patent ZL89106793.0 disclosed a synthetic method of high-silica mordenite which was to directly synthesize high-silica mordenite ($SiO_2/Al_2O_3$ molecular ratio was 15-30) in the presence of ammonia by using commercially available raw material such as sodium silicate, inorganic acid, inorganic alkali, aluminum salt and aluminates etc.. But it did not describe anything about the reaction performance of mordenite used as catalyst for the disproportionation and transalkylation of toluene and $C_9^+A$.

Chinese Patents CN1108214A, CN1108213A, and CN1105646A etc. described a synthesis method of β-zeolite with macro-pore structure used as a main active component of catalyst. But these patents did not describe anything about the reaction performance of catalysts when they were used in the disproportionation and transalkylation of toluene and $C_9^+A$. As compared with mordenite, due to the three-dimensional channel and macro pore structure, the amount of $C_{10}^+A$ formed at the end of the reaction was considerably large, so the selectivity of the desired products consequently dropped. Accordingly, β - zeolite had to be modified if it was used for the disproportionation and transalkylation of toluene and heavy aromatic hydrocarbons.

In the petrochemical industry, the key technology in all kinds of processes is the catalyst. It often takes place that a highly sophisticated process is always altered due to the industrialization of a new type of catalyst. The development and perfection of a petrochemical technical process will be attributed to the successful development of relevant catalysts and steady improvement of their performance. In order to enhance the processing capacity of the unit for the disproportionation and transalkylation of toluene and $C_9A$, and to reduce the energy consumption and feedstock consumption of the unit to reach a satisfied scale economics, various catalysts with high space velocity and high conversion for the disproportionation and transalkylation have been developed. But, as the conversion increases, the amount of $C_{10}^+A$ formed in the process also increases, so that the selectivity of the desired products decreases.

The object of the present invention is to provide a process for the disproportionation and transalkylation of toluene and $C_9^+A$. The present process overcomes such disadvantages of previous processes in the prior arts as the increase of light hydrocarbons in the system and the waste of resources, as well as the lower yield of benzene and $C_8A$ due to the fact that the processing capacity of the unit in the prior arts for the feedstock of aromatic hydrocarbons is lower and its processing capacity of $C_{10}^+A$ is poor or the said process only can be done by the means of dealkylation and lightening. The process of the present invention has a larger processing capacity of $C_{10}^+A$. It can effectively transform $C_{10}^+A$ into benzene and $C_8A$ when the process of disproportionation and transalkylation of toluene and $C_9^+A$ is carried out. The process of the present invention has a higher selectivity to the desired products under the condition of high space velocity and high conversion.

Another object of the present invention is to provide a catalyst used for the disproportionation and transalkylation of toluene and $C_9^+A$. The said catalyst has a high processing capacity of $C_{10}^+A$, in other words, the catalyst has high activity. In addition, the catalyst of the present invention can effectively transform $C_{10}^+A$ into benzene and $C_8A$ through the transalkylation.

## SUMMARY OF THE INVENTION

The present invention is a method of the disproportionation and transalkylation of toluene and $C_9^+A$. The method is carried out with the starting materials of the toluene and $C_9^+A$ in the presence of a modified catalyst of the invention in a fixed bed reactor at a high weight hourly space velocity.

The present invention is also a catalyst substantially made of hydrogen-form zeolite and promoters including molybdenum oxide, supported on the zeolite.

## DETAILED DESCRIPTION OF THE INVENTION

According to one respect of the present invention, a process for the disproportionation and transalkylation of toluene and heavy aromatic hydrocarbons including nine and more than nine carbon atoms comprises:

a) Toluene and $C_9^+A$ are used as the feedstock for the reaction wherein toluene: $C_9^+A$ is 95:5-5:95 by weight, preferably 90:10-30:70 by weight, more preferably 80:20- 40:60 by weight; the content of $C_{10}^+A$ in the feedstock for the reaction is 0-30% by weight, the said $C_9^+A$ is heavy aromatic hydrocarbons of nine and more than nine carbon atoms, and $C_{10}^+A$ is heavy aromatic hydrocarbons often and more than ten carbon atoms;

b) The weight hourly space velocity is 0.8-8.0 $hr^{-1}$, preferably 1.5-4.5 $hr^{-1}$;

c) The reaction temperature is 300-500°C, preferably 350-450°C;

d) In the presence of hydrogen, the reaction pressure is 1.0-5.0 MPa(G), preferably 2.0-3.5 MPa(G), the molar ratio of hydrogen to hydrocarbon is 1-10, preferably 3-7;

e) The catalyst for the disproportionation and transalkylation of toluene and $C_9^+A$ comprises by weight:

1) 20-90 parts of hydrogen-form zeolite having $SiO_2/Al_2O_3$ molar ratio 10-50, and the following components supported on the zeolite:

2) 0.05-10 parts of molybdenum oxide, preferably 0.1-5 parts;

3) 0-10 parts of at least one metal oxide selected from a group consisting of oxide of nickel or bismuth, preferably 0-5 parts, more preferably 0.1-5 parts;

4) 0-10 parts of at least one metal oxide selected from oxide of lanthanum, silver or rhenium, preferably 0.1-5 parts;

5) 10-60 parts of aluminum oxide as adhesive.

The content of $C_{10}^+A$ in the feedstock of the present invention can be up to 0-30% by weight, preferably 3-20% by weight, more preferably 5-15% by weight. Hydrogen-form zeolite in the catalyst is selected from mordenite, β -zeolite or the mixture thereof, preferably β -zeolite. The composition of the catalyst of the present invention comprises preferably bismuth oxide and/or nickel oxide and preferably, the amount thereof is 0-5 parts by weight, more preferably 0.1-5 parts by weight. The $SiO_2/Al_2O_3$ molar ratio in the hydrogen-form zeolite is preferably 15-35.

According to another respect of the present invention, the catalyst used in the process of the present invention comprises by weight:

1) 20-90 parts of hydrogen-form zeolite having $SiO_2/Al_2O_3$ molar ratio 10-50 and the following components supported on the zeolite:

2) 0.05-10 parts of molybdenum oxide, preferably 0.1-5 parts;

3) 0-10 parts of at least one metal oxide selected from a group consisting of oxide of nickel or bismuth, preferably 0-5 parts, more preferably 0.1-5 parts;

4) 0-10 parts of at least one metal oxide selected from oxide of lanthanum, silver or rhenium, preferably 0.1-5 parts;

5) 10-60 parts of aluminum oxide as adhesive.

The hydrogen-form zeolite used in the above-mentioned technical solution is selected from a group consisting of mordenite, β-zeolite or the mixture thereof, preferably β-zeolite. The $SiO_2/Al_2O_3$ molar ratio of hydrogen-form zeolite used is preferably within a range of 15-35. In the above-mentioned technical solution, the catalyst comprises, by weight, preferably 0-5 parts of bismuth oxide and/or nickel oxide, more preferably 0.1-5 parts of bismuth oxide and/or nickel oxide.

The catalyst used in the present invention is prepared and obtained by the following manner in which hydrogen-form zeolite and aluminum oxide are impregnated or mixed by the metal salts selectively used and then kneaded and extruded into strips, finally calcined at 350-600°C.

A stainless steel fixed bed reactor with 25mm inner diameter and 1200mm length is used to evaluate the catalyst' s reaction performance of the disproportionation and transalkylation of toluene and $C_9^+A$. The reactor has an electric heating system and its temperature is automatically controlled. Glass beads with 5mm diameter are filled in the bottom of the reactor as supporting materials, 20g of catalyst is filled in the reactor, and glass beads with 5mm diameter are filled in the upper section of the reactor for being used for preheating and vaporizing the feedstock. The toluene and $C_9^+A$ as the feedstock are mixed with hydrogen, then flow downward from the top through the layer of the catalyst bed, thereby the disproportionation and transalkylation are carried out to form benzene, $C_8A$ and small amount of alkanes such as methane, ethane, propane and butane, etc.

In carrying out the process of the present invention, the toluene and $C_9^+A$ obtained from an aromatic hydrocarbons complex in the petrochemical industry are used as the feedstock and the resultant evaluation data are calculated by using the following formulas:

$$\text{(Toluene and } C_9A\text{) conversion} = \frac{\text{Moles of Toluene and } C_9A \text{ converted in the reaction}}{\text{Moles of toluene and } C_9A \text{ pumped into the reactor}} \times 100\%$$

$$\text{(Benzene+}C_8A\text{) selectivity} = \frac{\text{Moles of benzene and } C_8A \text{ formed}}{\text{Moles of toluene and } C_9A \text{ converted in the reaction}} \times 100\%$$

$$C_{10}^+A \text{ conversion} = \frac{\text{Weight of } C_{10}^+A \text{ converted in the reaction}}{\text{Weight of } C_{10}^+A \text{ pumped into the reactor}} \times 100\%$$

The present invention will be further illustrated through the following non-limiting examples.

Examples 1-5

66.7g of ammonium-form mordenite powder ($Na_2O$ content: less than 0.15% (wt), $SiO_2/Al_2O_3$ molar ratio: 15-35), and 57.1g of $\alpha$-$Al_2O_3 \cdot H_2O$ ($Na_2O$ content: less than 0.15% (wt)) are mixed thoroughly, then the mixture is impregnated in the aqueous solution of ammonium molybdate (chemically pure). A diluted solution of nitric acid (chemically pure) is added into the impregnated powder. The resultant mixture is kneaded thoroughly, extruded into strips, calcined to form catalyst. The catalysts A, B, C, D, E in which the content of molybdenum oxide is respectively 0.1%, 1.0%, 2.0%, 3.0%, and 5.0%(wt) are prepared by the similar method in which the different content of ammonium molybdate is used.

Examples 6-10

The catalysts A1, B1, C1, D1, E1 in which the content of molybdenum oxide is respectively 0.1%, 1.0%, 2.0%, 3.0%, 5.0% (wt) are prepared in the same manner as in Example 1 except that mordenite is replaced by $\beta$ -zeolite ($Na_2O$ content: less than 0.15%(wt), $SiO_2/Al_2O_3$ molar ratio: 15-40).

Comparative Example 1

The catalyst G is prepared in the same manner as in Example 1 except that ammonium molybdate is not added.

Example 11

Catalyst evaluation of the molybdenum-contained catalysts A-E, A1-E1 prepared in the same manner as in Example 1-10 and the catalyst G prepared in the same manner as in comparative Example 1 is conducted in a fixed bed reactor to determine their activity for the disproportionation and transalkylation of toluene and $C_9^+A$. In the evaluation, 20g of catalyst is loaded into the reactor, the weight hourly space velocity is $2.5hr^{-1}$, the reaction temperature is 390°C, the reaction pressure is 3.0MPa(G), the molar ratio of hydrogen to hydrocarbon is 3.5, toluene: $C_9^+A$ in the feedstock is 60:40(wt/wt), the said $C_9^+A$ contains 12.5% of $C_{10}^+A$ (wt). The $C_9A$ in the feedstock comprises (wt): 3.45% of propylbenzene, 33.14% of methylethylbezene, 63.40% of trimethylbenzene. The said $C_{10}^+A$ comprises (weight): 3.33% of diethylbenzene, 26.96% of dimethylethyl benzene, 2.32% of methyl propylbenzene, 28.84% of tetramethylbenzene, 14.49% of methyl naphthalene, 11.16% of dimethyl-naphthalene, 12.90% of other $C_{11}^+A$. The evaluation results are summarized in Table 1:

Table 1.

| Evaluation Results | | | | | | |
|---|---|---|---|---|---|---|
| Catalyst | Molecular sieve | $SiO_2/Al_2O_3$ molar ratio | $MoO_3$% (wt) | (Toluene $+C_9A$) conversion % | $C_{10}{}^+A$ conversio n % | (Benzene+$C_8A$) selectivity % |
| A | Mordenite | 33.0 | 0.1 | 30.2 | 22.8 | 98.2 |
| B | | 22.3 | 1.0 | 35.6 | 24.6 | 97.8 |
| C | | 15.0 | 2.0 | 28.4 | 24.2 | 98.5 |
| D | | 25.4 | 3.0 | 41.8 | 22.6 | 96.4 |
| E | | 22.3 | 5.0 | 46.0 | 30.4 | 95.3 |
| A1 | β-zeolite | 28.0 | 0.1 | 29.6 | 12.2 | 97.7 |
| B1 | | 15.6 | 1.0 | 34.3 | 14.0 | 97.6 |
| C1 | | 24.6 | 2.0 | 40.2 | 14.2 | 96.8 |
| D1 | | 31.4 | 3.0 | 42.4 | 18.0 | 96.1 |
| E1 | | 28.0 | 5.0 | 46.8 | 22.3 | 94.8 |
| G | Mordenite | 33.0 | 0 | 24.5 | / | 97.5 |

The catalyst of molybdenum-contained mordenite or β-zeolite prepared by the present invention, no matter whether the content of molybdenum is high or low, has a higher activity as compared with the catalyst which does not contain molybdenum.

Example 12

The catalyst H, L, N, O, R and S are prepared in the same manner as in Example 1 or 9, except that the ratio of molecular sieve to aluminum oxide is changed. The catalyst H containing 1.0% (wt) of $MoO_3$ and 0.5% (wt) of $Bi_2O_3$ is prepared in the same manner as in Example 1, and the diluted solution of ammonium molybdate in nitric acid in Example 1 is replaced by diluted solution of ammonium molybdate and bismuth nitrate in nitric acid. The similar process is used to prepare respectively catalyst L, N, O, R and S, each containing one or more metals selected from bismuth, nickel, lanthanum, silver and/or rhenium. The activity of such catalysts is evaluated by using the feedstock with the same composition in the same equipment at same reaction conditions as in Example 11. The results are shown in Table 2.

### Table2.

#### The evaluating activity of catalysts

| Composition (wt %) \ Number | H | L | N | O | R | S |
|---|---|---|---|---|---|---|
| $MoO_3$ | 1.0 | 4.0 | 3.0 | 1.0 | 5.0 | 5.0 |
| $La_2O_3$ | | | | 0.5 | | |
| $Bi_2O_3$ | 0.5 | 1.5 | 0.5 | | | |
| NiO | | | 0.5 | 1.0 | | |
| $Ag_2O$ | | | | | 1.0 | |
| ReO | | | | | | 0.3 |
| Zeolite | M* | β ** | β | M | β | β |
| Molecular seive/$Al_2O_3$ (wt/wt) | 50/50 | 30/70 | 70/30 | 70/30 | 60/40 | 40/60 |
| (Toluene+$C_9A$) conversion % | 40.8 | 44.2 | 47.8 | 42.7 | 43.6 | 42.0 |
| (Benzene+$C_8A$) selectivity% | 96.8 | 95.4 | 93.6 | 95.8 | 95.6 | 95.9 |

*M represents mordenite; ** β represents β -zeolite.

The evaluation results indicate that the catalyst containing one or more metal oxides of molybdenum, bismuth, lanthanum, nickel, silver and/or rhenium prepared by the present invention has fairly good catalystic activity for the disproportionation and transalkylation of toluene and $C_9^+A$.

Examples 13-22

The similar catalytic evaluation is conducted by using the catalysts as in above-mentioned examples and the same the reaction feedstock as in Example 11, properly adjusting the content of toluene and $C_9^+A$ composition, and adding indane (IND), changing other evaluating conditions. The reaction results are summarized in Table 3 and Table 4.

Table 3.

| The process technical indexes of the present invention under different the reaction conditions | | | | | |
|---|---|---|---|---|---|
| Example | 13 | 14 | 15 | 16 | 17 |
| Catalyst | C1 | D | E | E1 | N |
| Composition of the reaction feedstock (wt%) | | | | | |
| Toluene | 90.0 | 75.0 | 60.0 | 56.0 | 40.0 |
| $C_9A$ | 5.0 | 10.0 | 36.3 | 35.0 | 48.0 |
| IND | 0.4 | 0.8 | 0.6 | 0.6 | 0.7 |
| $C_{10}^+A$ | 4.6 | 14.2 | 3.1 | 8.4 | 11.3 |
| Total | 100.0 | 100.0 | 100.0 | 100.0 | 100.0 |
| Weight hourly space velocity (whsv) ,$hr^{-1}$ | 4.5 | 4.0 | 3.0 | 2.5 | 1.5 |
| The reaction pressure, MPa(G) | 3.5 | 2.5 | 2.8 | 3.0 | 2.1 |
| The molar ratio of hydrogen to hydrocarbon, mol/mol | 3.1 | 4.0 | 5.0 | 5.5 | 8.0 |
| The reaction temperature, °C | 450 | 440 | 380 | 390 | 330 |
| The reaction results, % (Toluene+$C_9A$) conversion | 40.0 | 40.2 | 42.0 | 46.0 | 43.3 |

Table 3.  (continued)

| Example | 13 | 14 | 15 | 16 | 17 |
|---|---|---|---|---|---|
| $C_{10}^+A$ conversion | 40.1 | 35.0 | 6.1 | 32.0 | 40.0 |
| (Benzene+$C_8A$) selectivity | 97.0 | 99.5 | 95.1 | 96.0 | 98.0 |

Table4.

| The process technical indexes of the present invention under different the reaction conditions | | | | | |
|---|---|---|---|---|---|
| Example | 18 | 19 | 20 | 21 | 22 |
| Catalyst | H | L | R | S | O |
| Composition of the reaction feedstock (wt%) | | | | | |
| Toluene | 63.0 | 64.6 | 60.0 | 54.6 | 55.0 |
| $C_9A$ | 36.6 | 30.0 | 32.5 | 38.0 | 37.1 |
| IND | 0.4 | 0.4 | 0.5 | 0.4 | 0.8 |
| $C_{10}^+A$ | / | 5.0 | 7.0 | 7.0 | 7.1 |
| Total | 100.0 | 100.0 | 100.0 | 100.0 | 100.0 |
| Weight hourly space velocity (whsv), $hr^{-1}$ | 2.5 | 2.5 | 2.0 | 1.0 | 3.0 |
| The reaction pressure, MPa(G) | 3.0 | 2.6 | 3.0 | 1.5 | 3.0 |
| The molar ratio of hydrogen to hydrocarbon, mol/mol | 3.5 | 3.5 | 4.2 | 8.5 | 5.5 |
| The reaction temperature, °C | 390 | 395 | 400 | 340 | 410 |
| The reaction results,% | | | | | |
| (Toluene+$C_9A$) conversion | 42.0 | 45.1 | 46.2 | 40.4 | 45.5 |
| $C_{10}^+A$ conversion | / | 30.2 | 42.3 | 40.5 | 40.1 |
| (Benzene+$C_8A$) selectivity | 94.8 | 95.0 | 97.5 | 98.5 | 97.4 |

Example 23

The disproportionation and transalkylation of toluene and $C_9^+A$ are carried out by using the catalyst E prepared in example 5 and the feedstock with different content of $C_{10}^+A$ wherein the ratio of toluene to $C_9^+A$ and the composition of $C_9A$ and $C_{10}^+A$ in the feedstock are the same as in Example 11, to evaluate the processing capacity of $C_{10}^+A$. The rest of conditions used in the said evaluation process are the same as in Example 11. The evaluating results are shown in Table 5.

Table 5.

| Evaluation Results | | | | |
|---|---|---|---|---|
| The Content of $C_{10}^+A$ in $C_9^+A$ wt% | (Toluene+$C_9A$) Conversion % | $C_{10}^+A$ Conversion % | (Benzene+$C_8A$) Selectivity % | Light Hydrocarbons* g |
| 12.5 | 46.0 | 30.4 | 95.3 | 4.7 |
| 17.5 | 44.5 | 36.6 | 97.0 | 4.7 |
| 25.0 | 43.2 | 38.0 | 99.5 | 4.8 |

Annotation*: the quantity of $C_5$ hydrocarbons and hydrocarbons of less than five carbon atoms in 100g of reaction effluent.

The data in table 5 indicate that the process of this invention can effectively process a feedstock with higher content of $C_{10}^+A$. It can be seen, from table 5, that as the content of $C_{10}^+A$ in the feedstock increases, the selectivity of desired products (benzene and $C_8A$) increases, but the quantity of light hydrocarbons is almost the same. It means that the converted $C_{10}^+A$ are effectively transformed into the desired products of benzene and $C_8A$ etc. mainly through the process of transalkylation.

Due to the excellent performance of the catalyst, the process of the present invention can be used to produce desired products with higher content of benzene and $C_8A$ since it has a function of transalkylation of toluene and $C_9^+A$. In addition, benzene and $C_8A$ are also greatly formed through the disproportionation of toluene itself. Therefore, in the process of the present invention, the disproportionation and transalkylation are the main the reactions other than the hydrodealkylation, so that it avoids the formation of a large amount of light hydrocarbons and avoids the waste of the feedstock.

Because the catalyst used in the present invention has not only the function of disproportionation but also the function of transalkylation, it breaks through the limitation that only pure toluene is capable to be used as the reaction feedstock. In addition, in the technical solution of the present invention, molybdenum and other active components, especially molybdenum oxide, nickel oxide and/or bismuth oxide and other no-essential oxides selected for lanthanum, silver or rhenium oxides are supported on the zeolite, especially mordenite or $\beta$-zeolite. This makes the prepared catalysts have high catalytic activity, so that the content of $C_{10}^+A$ in the feedstock can reach up to 30%(wt), while the selectivity to (benzene +$C_8A$) in the products can reach up to 99.5%. The present invention makes $C_{10}^+A$ be effectively transformed into benzene and $C_8A$, thereby a better result can be achieved. In the catalyst of the present invention, the total amount of acid is effectively raised and the acidity is adjusted by the means of adding molybdenum oxide and nickel oxide or/and bismuth oxide supported on mordenite, $\beta$-zeolite or the mixture thereof. It not only improves the catalytic activity but also prevents the side the reaction from taking place, thereby the processing capacity of the catalyst and the selectivity to the desired products are substantially enhanced. The process of this present invention has excellent technical and economic indexes and has high selectivity to desired products under high space velocity and high conversion. No doubt, the present invention can be expected to be industrialized successfully.

While the invention has been described with reference to specific embodiments, it will be apparent that numerous variations, modifications and alternative embodiments of the invention are possible, and accordingly all such variations, modifications, and alternative embodiments are to be regarded as being within the spirit and scope of the present invention as claimed.

**Claims**

1. A process for the disproportionation and transalkylation of toluene and heavy aromatic hydrocarbons of nine and more than nine carbon atoms comprises:

   a) Toluene and $C_9^+A$ are used as the feedstock for the reaction wherein the ratio of toluene to $C_9^+A$ is 95:5-5:95 by weight, the content of $C_{10}^+A$ in the feedstock for the reaction is 0-30% by weight, the said $C_9^+A$ is heavy aromatic hydrocarbons of nine and more than nine carbon atoms, and $C_{10}^+A$ is heavy aromatic hydrocarbons often and more than ten carbon atoms;
   b) The weight hourly space velocity is 0.8-8.0 hr$^{-1}$;
   c) The reaction temperature is 300-500°C;
   d) In the presence of hydrogen, the reaction pressure is 1.0-5.0 MPa(G) and the molar ratio of hydrogen to hydrocarbon is 1-10;
   e)The catalyst for the disproportionation and transalkylation of toluene and $C_9^+A$ comprises by weight:

   1) 20-90 parts of hydrogen-form zeolite having $SiO_2/Al_2O_3$ molar ratio 10-50, and the following components supported on the zeolite:
   2) 0.05-10 parts of molybdenum oxide;
   3) 0-10 parts of at least one metal oxide selected from a group consisting of oxide of nickel or bismuth;
   4) 0-10 parts of at least one metal oxide selected from oxide of lanthanum, silver or rhenium;
   5) 10-60 parts of aluminum oxide as adhesive.

2. The process of claim 1, wherein

   a) The ratio of toluene/$C_9^+A$ is 90:10-30:70 by weight, and the content of $C_{10}^+A$ in the reaction feedstock is 3-20% by weight;
   b) The weight hourly space velocity is 1.5-4.5 hr$^{-1}$;
   c) The reaction temperature is 350-450°C;
   d) The reaction pressure is 2.0-3.5 MPa(G); The molar ratio of hydrogen to hydrocarbon is 3-7.

3. The process of claim 2, wherein the ratio of toluene to $C_9^+A$ is 80:20-40:60 by weight, and the content of $C_{10}^+A$ in the reaction feedstock is 5-15% by weight.

4. The process of claim 1, wherein the hydrogen-form zeolite of the said catalyst is selected from a group consisting of mordenite, $\beta$-zeolite or the mixture thereof.

**5.** The process of claim 4, wherein the hydrogen-form zeolite is $\beta$-zeolite.

**6.** The process of claim 1, wherein the amount of the molybdenum oxide contained in the catalyst is 0.1-5 parts by weight.

**7.** The process of claim 1, wherein the catalyst contains 0-5 parts by weight of at least one component selected from a group consisting of oxide of nickel or bismuth.

**8.** The process of claim 7, wherein the catalyst contains 0.1-5 parts by weight of at least one component selected from a group consisting of oxide of nickel or bismuth.

**9.** The process of claim 1, wherein the catalyst contains 0-5 parts by weight of nickel oxide.

**10.** The process of claim 9, wherein the catalyst contains 0.1-5 parts by weight of nickel oxide.

**11.** The process of claim 1, wherein the catalyst contains 0-5 parts by weight of bismuth oxide.

**12.** The process of claim 11, wherein the catalyst contains 0.1-5 parts by weight of bismuth oxide.

**13.** The process of claim 1, wherein the catalyst contains 0.1-5 parts by weight of at least one component selected from a group consisting of oxide of lanthanum, silver or rhenium.

**14.** A catalyst used for any one of the processes of claiml-13, comprising

    a) 20-90 parts by weight of hydrogen-form zeolite having $SiO_2/Al_2O_3$ molar ratio 10-50, and the following components supported on the zeolite:
    b) 0.05-10 parts by weight of molybdenum oxide;
    c) 0-10 parts by weight of at least one metal oxide selected from a group consisting of oxide of nickel or bismuth;
    d) 0-10 parts by weight of at least one metal oxide selected from oxide of lanthanum, silver or rhenium;
    e) 10-60 parts by weight of aluminum oxide as adhesive.

**15.** The catalyst of claim 14, wherein the $SiO_2/Al_2O_3$ molar ratio of the hydrogen-form zeolite in the catalyst is 15-35.

**16.** The catalyst of claim 14, wherein the hydrogen-form zeolite is selected from a group consisting of mordenite, $\beta$-zeolite or the mixture thereof.

**17.** The catalyst of claim 16, wherein the hydrogen-form zeolite is $\beta$-zeolite.

**18.** The catalyst of claim 14, wherein the content of molybdenum oxide in the catalyst is 0.1-5 parts by weight.

**19.** The catalyst of claiml4, wherein it contains 0-5 parts by weight of at least one component selected from a group consisting of oxide of nickel or bismuth.

**20.** The catalyst of claiml4, wherein it contains 0.1-5 parts by weight of at least one component selected from a group consisting of oxide of nickel or bismuth.

**21.** The catalyst of claim 14, wherein it contains 0-5 parts by weight of nickel oxide.

**22.** The catalyst of claim 21, wherein it contains 0.1-5 parts by weight of nickel oxide.

**23.** The catalyst of claims 14, wherein it contains 0-5 parts by weight of bismuth oxide.

**24.** The catalyst of claim 23, wherein it contains 0.1-5 parts by weight of bismuth oxide.

**25.** The catalyst of claim 14, wherein it contains 0.1-5 parts by weight of at least one component selected from a group consisting of oxide of lanthanum, silver or rhenium.

EP 1 106 592 A1

# INTERNATIONAL SEARCH REPORT

International application No.

**PCT/CN00/00187**

### A. CLASSIFICATION OF SUBJECT MATTER

**IPC 7 C07C6/12 C07C15/06 C07C15/08 B01J29/16 B01J29/26**

According to International Patent Classification (IPC) or to both national classification and IPC

### B. FIELDS SEARCHED

Minimum documentation searched (classification system followed by classification symbols)

**IPC 7 C07C , B01J**

Documentation searched other than minimum documentation to the extent that such documents are included in the fields searched

Electronic data base consulted during the international search (name of data base and, where practicable, search terms used)

**CNPAT EOQUE**

### C. DOCUMENTS CONSIDERED TO BE RELEVANT

| Category* | Citation of document, with indication, where appropriate, of the relevant passages | Relevant to claim No. |
|---|---|---|
| A | CN1217369 （China Petro-Chemical Corporation et.al） | 1,14 |
| | 26.05.99 Whole document | |
| A | US 5,866,741 （An-hsang Wu et.al） | 1,14 |
| | 02.02.1999 Whole document | |
| A | US3,551,509 （Owen H.Thomas et al） | 1 |
| | 29.12.1970 Whole document | |

☐ Further documents are listed in the continuation of Box C. ☒ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance: the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" | document of particular relevance: the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date | "&" | document member of the same patent family |

| Date of the actual completion of the international search | Date of mailing of the international search report |
|---|---|
| 03.Aug.2000(03.08.00) | **1 0 AUG 2000** **(1 0. 0 8. 0 0)** |

| Name and mailing address of the ISA/CN | Authorized officer |
|---|---|
| 6 Xitucheng Rd.. Jimen Bridge. Haidian District, 100088 Beijing, China | Hou Yue |
| Facsimile No. 86-10-62019451 | Telephone No. 86-10-62093897 |

Form PCT/ISA /210 (second sheet) (July 1998)

12

## INTERNATIONAL SEARCH REPORT
Information on patent family members

| International application No. |
| --- |
| PCT/CN00/00187 |

| Patent document cited in search report | Publication date | Patent family member(s) | Publication date |
| --- | --- | --- | --- |
| CN-A-1217369 | 26-05-99 | JP-A-1105481 | 02-03-99 |
| | | EP-A-0884103 | 16-12-98 |

Form PCT/ISA /210 (patent family annex) (July 1998)

13